# EUROPEAN PATENT SPECIFICATION

(11) **EP 4 456 772 B1**
(45) Date of publication and mention of the grant of the patent: **25.11.2026**
(21) Application number: 22913325.1
(22) Date of filing: 22.07.2022
(51) Int. Cl.: A47K 7/04, A45D 34/00, A61N 5/06, A41H 33/00

(54) **HANDHELD SKIN CLEANSING DEVICE**
TRAGBARE HAUTREINIGUNGSVORRICHTUNG
DISPOSITIF PORTATIF DE NETTOYAGE DE LA PEAU

(30) Priority: 31.12.2021 CN 202111659125
(43) Date of publication of application: 06.11.2024
(73) Proprietor: Beauty Biosciences LLC, Dallas, Texas 75219 (US)
(72) Inventor: GONG, Maoxiang, Shenzhen, Guangdong 518029 (CN)
(74) Representative: CMS Cameron McKenna Nabarro Olswang LLP
(86) International application number: PCT/CN2022/107540
(87) International publication number: WO 2023/124036

(56) References cited:
- WO-A1-2012/033265
- CN-U- 209 500 518
- CN-U- 210 277 005
- CN-U- 210 277 005
- CN-U- 210 353 637
- CN-U- 211 212 846
- CN-U- 214 073 086
- CN-U- 214 073 086
- CN-U- 214 760 818
- KR-A- 20200 134 792
- TW-U- M 611 745
- TW-U- M 611 745

## Description

This application claims the benefit of and priority to China Patent Application No. 202111659125.1, filed December 31, 2021.

### BACKGROUND

Existing handheld devices that utilize a pump and fluid to cleanse the skin suffer several drawbacks. For example, may such devices are susceptible to ingesting fluid into the pump under various circumstances, which can damage the pump and potentially require replacement. As another example, many such devices rely provide only fluid-based cleansing treatments and thus provide limited cleansing or require the user to apply other treatments separately, oftentimes using a separate device. Still further, many such devices can become unhygienic and thereby pose a potential infection risk or negative user experience. An example of a handheld skin cleansing device is known from CN 210 277 005 U. Accordingly, there is a need for new and/or improved handheld skin devices.

### SUMMARY

The present disclosure is directed to a handheld skin cleansing device including a first conduit providing fluid communication between a treatment container and a first opening in a tip of the device; a second conduit providing fluid communication between a second opening in the tip and a waste container of the device, the waste container being fixedly attached to a handheld body of the device and having an opening sealed by removable cap; a pump housed within the handheld body and in fluid communication with the waste container, the pump configured to form a pressure differential between the waste container and the second opening in the tip so as to direct fluid from the treatment container, to the tip, and into the waste container via the first and second conduits when the tip is applied to skin of a user; and a valve configured to open to permit fluid communication between the waste container and the pump in response to suction forces generated by operation of the pump, and to close to obstruct fluid communication between the waste container and the pump in the absence of the suction forces when the pump is not being operated.

The valve, in various embodiments, may include a piston configured to move between a first position and a second position and wherein, in the first position, the piston does not obstruct fluid communication between the waste container and the pump and wherein, in the second position, the piston obstructs fluid communication between the waste container and the pump; and a biasing member coupled to the piston, the biasing member being configured to permit the piston to move to the first position in response to the suction forces generated by operation of the pump, and to bias the piston into the second position in the absence of the suction forces when the pump is not being operated. The removable cap, in various embodiments, may be configured to be opened to allow the waste fluid to be drained from the waste container through the opening and to introduce a cleaning fluid into the waste container, and the valve may close in the absence of the suction forces when the pump is not being operated, thereby allowing the device to be inverted to facilitate the introduction of the cleaning fluid into the waste container through the opening without the cleaning fluid draining into the pump. The handheld skin cleansing device, in various embodiments, may further include a second valve situated within the second conduit, the second valve configured to obstruct the flow of fluid therethrough in a direction towards the tip when a pressure differential across the second conduit is released.

The handheld skin cleansing device, in various embodiments, may further include a pump protection system configured to turn the pump off upon detecting any one or more of the following: (i) that fluid in the waste container reaches a predetermined level, (ii) that fluid in the treatment container reaches a predetermined level corresponding with having dispensed enough fluid for the fluid to reach a predetermined level within the waste container, and (iii) that there is moisture upstream of the pump. The fluid-level-based pump protection system, in an embodiment, may include a hall effect float sensor. **In** another embodiment, the fluid-level-based pump protection system may include a sensor having three or more conductive pins extending an equal distance into the waste container and arranged about a perimeter of an interior of the waste container, the pins being configured to detect when waste fluid contacts at least two of the pins simultaneously.

The handheld skin cleansing device, in various embodiments, may further include a pump protection system configured to detect that an angle of the device has exceeded a predetermined threshold and to turn off the pump in response. The tilt-based pump protection system, in an embodiment, may include a tilt angle sensor.

The handheld skin cleansing device, in various embodiments, may further include a trap having a tortuous conduit extending between a first opening and a second opening, the first opening being in fluid communication with an interior of the treatment container and the second opening being in fluid communication with an area outside of the treatment container.

**In** another aspect, the present disclosure is directed to a handheld skin cleansing device including a first conduit providing fluid communication between a treatment container and a first opening in a tip of the device; a second conduit providing fluid communication between a second opening in the tip and a waste container of the device; a pump housed within the handheld body and in fluid communication with the waste container, the pump configured to form a pressure differential between the waste container and the second opening in the tip so as to direct fluid from the treatment container, to the tip, and into the waste container via the first and second conduits when the tip is applied to skin of a user; and a pump protection system configured to turn the pump off upon detecting at least one of the following: (i) that fluid in the waste container reaches a predetermined level or (ii) that fluid in the treatment container reaches a predetermined level corresponding with having dispensed enough fluid for the fluid to reach a predetermined level within the waste container, the pump protection system comprising a sensor having three or more conductive pins extending an equal distance into the waste container or the treatment container, respectively, and arranged about a perimeter of an interior of the waste container or the treatment container, respectively, the pins being configured to detect when fluid in the waste container or the treatment container, respectively, contacts at least two of the pins simultaneously.

**In** various embodiments, the three or more conductive pins may extend into the waste container from an upper portion of the waste container towards a lower portion of the waste container, and the distance by which the three or more pins extend into the waste container may be configured to place a distal end of each pin at a location corresponding with the predetermined level of fluid. Detecting contact of the fluid with at least two of the pins simultaneously may indicate that the fluid in the waste container has reached the predetermined fluid level.

**In** various embodiments, the three or more conductive pins may extend into the treatment container from an upper portion of the treatment container towards a lower portion of the treatment container, and the distance by which the three or more pins extend into the treatment container may be configured to place a distal end of each pin at a location corresponding with having dispensed enough fluid for the fluid to reach a predetermined level within the waste container. Detecting a lack of contact of the fluid with at least two of the pins simultaneously may indicate that the fluid in the treatment container has reached a predetermined level corresponding with having dispensed enough fluid for the fluid to reach a predetermined level within the waste container.

The arrangement of the pins about a perimeter of the interior of the waste container or the treatment container, in various embodiments, may permit the sensor to detect conditions (i) or (ii) regardless of an angle at which the device is tilted. The pump protection system, in various embodiments, may also be configured to prevent the pump from being turned back on until detecting that the waste container has been opened, detached, or otherwise emptied.

The handheld skin cleansing device, in various embodiments, may further include a pump protection system configured to detect that an angle of the device has exceeded a predetermined threshold and to turn off the pump in response. The pump protection system, in an embodiment, may comprise a tilt angle sensor.

The handheld skin cleansing device, in various embodiments, may further include a valve configured to open to permit fluid communication between the waste container and the pump in response to suction forces generated by operation of the pump, and to close to obstruct fluid communication between the waste container and the pump in the absence of the suction forces when the pump is not being operated.

The handheld skin cleansing device, in various embodiments, may further include a valve situated within the second conduit, the valve configured to obstruct the flow of fluid therethrough in a direction towards the tip when a pressure differential across the second conduit is released.

The handheld skin cleansing device, in various embodiments, may further include a trap having a tortuous conduit extending between a first opening and a second opening, the first opening being in fluid communication with an interior of the treatment container and the second opening being in fluid communication with an area outside of the treatment container.

In yet another aspect, the present disclosure is directed to a handheld skin cleansing device including a first conduit providing fluid communication between a treatment container and a first opening in a tip of the device; a second conduit providing fluid communication between a second opening in the tip and a waste container of the device; a pump housed within the handheld body and in fluid communication with the waste container, the pump configured to form a pressure differential between the waste container and the second opening in the tip so as to direct fluid from the treatment container, to the tip, and into the waste container via the first and second conduits when the tip is applied to skin of a user; and a trap having a tortuous conduit extending between a first opening and a second opening, the first opening being in fluid communication with an interior of the treatment container and the second opening being in fluid communication with an area outside of the treatment container. In various embodiments, the area outside of the treatment container is a hollow interior portion of the handheld body. The trap, in various embodiments, may include a planar body and the tortuous conduit may include a hollow channel extending within the planar body in a planar direction.

In still another aspect, the present disclosure is directed to a handheld skin cleansing device including a tip and a body portion having a distal end on which the tip is situated or integrally formed. The tip may have an outer surface; a first opening through which a treatment fluid is delivered to the outer surface; and a second opening through which the treatment fluid is withdrawn from the outer surface. The body portion may have a pump configured to deliver and withdraw the treatment fluid to and from the outer surface, respectively, and a light source at a distal end of the body portion, the light source configured to emit light towards a user's skin proximate the outer surface. Operation of the light source may be selectively controllable via an interface on the main body portion.

The light source, in various embodiments, may be configured to emit light in a wavelength configured to provide a therapeutic, anti-bacterial, or other treatment effect. A second light source may be included, wherein the first light source may be configured to emit light in a first wavelength, and wherein the second light source is configured to emit light in a second wavelength.

In a further aspect, the present disclosure is directed to a tip for a handheld skin cleansing device, the tip including an outer surface; a first opening through which a treatment fluid is delivered to the outer surface; a second opening through which the treatment fluid is withdrawn from the outer surface; and a light source configured to emit light towards a user's skin proximate the outer surface. Operation of the light source may be selectively controllable via an interface on the main body portion.

The light source, in various embodiments, may be configured to emit light in a wavelength configured to provide a therapeutic, anti-bacterial, or other treatment effect. A second light source may be included, wherein the first light source may be configured to emit light in a first wavelength, and wherein the second light source is configured to emit light in a second wavelength.

### BRIEF DESCRIPTION OF THE DRAWINGS

Illustrative, non-limiting example embodiments will be more clearly understood from the following detailed description taken in conjunction with the accompanying drawings.
FIG. 1 illustrates a representative handheld skin cleansing device 100, in accordance with an embodiment of the present disclosure;
FIG. 2A, FIG. 2B, and FIG. 2C show device 100 with bottom case 52 and transparent case 51 removed, revealing a first container 46 and a second container 47, in accordance with an embodiment of the present disclosure;
FIG. 3 illustrates handheld skin cleansing device 100 with top case 9 further removed, revealing a pump 17, in accordance with an embodiment of the present disclosure;
FIG. 4 illustrates handheld skin cleansing device 100 with pump holders 16 and 26 removed, revealing a first conduit 24 connecting treatment container 46 and tip 1, in accordance with an embodiment of the present disclosure;
FIG. 5 is a cutaway view of an upper portion of handheld skin cleansing device 100 comprising tip 1 and pump 17 and illustrates representative connections of first conduit 24 and a second conduit 23 with tip 1, in accordance with an embodiment of the present disclosure;
FIG. 6 is a side view of an interior of handheld skin cleansing device 100, in accordance with an embodiment of the present disclosure;
FIG. 7 is a pseudo-schematic view illustrating fluid connections amongst various components of handheld skin cleansing device 100, in accordance with an embodiment of the present disclosure;
FIG. 8 is a pseudo-schematic view illustrating electrical connections amongst various components of handheld skin cleansing device 100, in accordance with an embodiment of the present disclosure;
FIG. 9A and FIG. 9B illustrate representative embodiments of tip 1 of handheld skin cleansing device 100, in accordance with an embodiment of the present disclosure;
FIG. 9C (iso front view) and FIG. 9D (schematic) illustrate the tip 1 of FIG. 9A with light sources 90 on a front face thereof, in accordance with an embodiment of the present disclosure;
FIG. 9E (iso front view) and FIG. 9F (schematic) illustrate the tip 1 of FIG. 9B with light sources 90 on a front face thereof, in accordance with an embodiment of the present disclosure;
FIG. 9G (iso front view) and FIG. 9H (schematic) illustrate the tip 1 of FIG. 9A with light sources 90 the distal end of the main body portion of device 100 behind tip 1, in accordance with an embodiment of the present disclosure;
FIG. 9I (iso front view) and FIG. 9J (schematic) illustrate the tip 1 of FIG. 9B with light sources 90 the distal end of the main body portion of device 100 behind tip 1, in accordance with an embodiment of the present disclosure;
FIG. 9K (front view, tip 1 removed), FIG. 9L (side view, tip 1 removed), and FIG. 9M (front view, transparent tip 1 attached) illustrate a representative configuration in which light source(s) 90 are located at the distal end of the main body portion of device 100 behind tip 1 and where tip 1 is transparent to allow light emitted from the light source(s) 90 to shine through to illuminate the user's skin in operation, in accordance with an embodiment of the present disclosure;
FIG. 9N and FIG. 9O illustrate a representative embodiment of tip 1 configured as a protective cap, in accordance with an embodiment of the present disclosure;
FIG. 10, FIG. 11, and FIG. 12 illustrate a representative pump protection system 200 configured to protect pump 17 from ingesting waste fluid from waste container 47 while pump 17 is turned off, in accordance with an embodiment of the present disclosure;
FIG. 13, FIG. 14, and FIG. 15 illustrate a representative pump protection system 300 configured to protect pump 17 from ingesting waste fluid from waste container 47 in various embodiments while pump 17 is turned on, such as when waste container 47 is overfilled, in accordance with an embodiment of the present disclosure;
FIG. 16 is an exploded view of the representative skin cleansing device 100, in accordance with an embodiment of the present disclosure;
FIG. 17, FIG. 18, FIG. 19, FIG. 20A, FIG. 20B, and FIG. 21, illustrate a representative embodiment of a pump protection system 600, in accordance with an embodiment of the present disclosure;
FIG. 22 is a schematic view of device 100 illustrating a head leakage valve 70 positioned within conduit 23 and configured to prevent or mitigate leakage of fluid out of tip 1 when device 100 is inverted for cleaning, in accordance with an embodiment of the present disclosure;
FIG. 23, FIG. 24, and FIG. 25 illustrate a trap 80 configured to prevent or mitigate leakage of treatment fluid out of the top of treatment container 46 when treatment container 46 is being connected to device 100, in accordance with an embodiment of the present disclosure; and
FIG. 26 and FIG. 27 show a hinged removable end cap 50 in a closed and open position, respectively, in accordance with an embodiment of the present disclosure.

### DETAILED DESCRIPTION

### Handheld Skin Cleansing Device 100

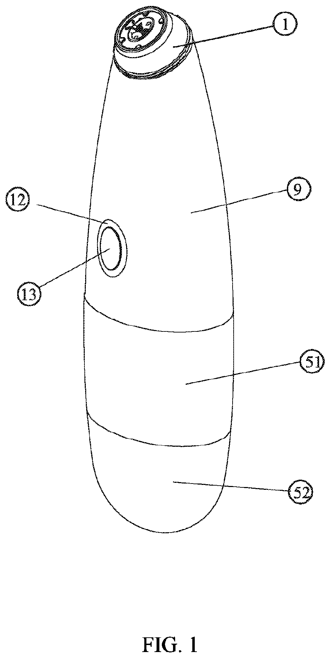

FIG. 1 illustrates a representative handheld skin cleansing device 100. Generally speaking, handheld skin cleansing device may include a body portion (shown here as including top case 9, transparent case 51, and bottom case 52) configured to be held in a user's hand, a tip 1 configured to contact a user's skin, and a control interface (shown here as silicon button 12 and button key 13) for operating device 100.

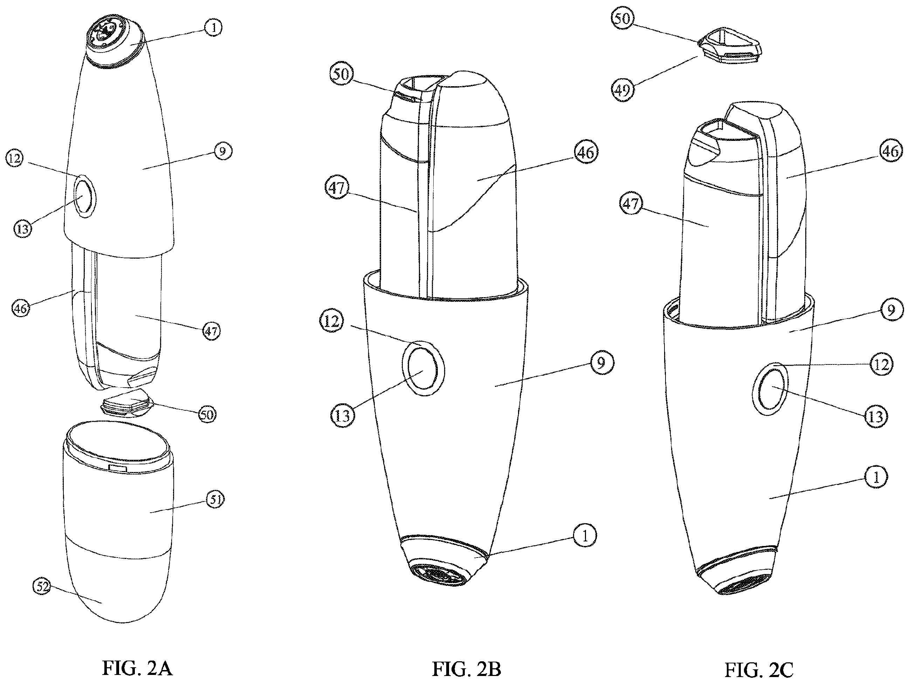

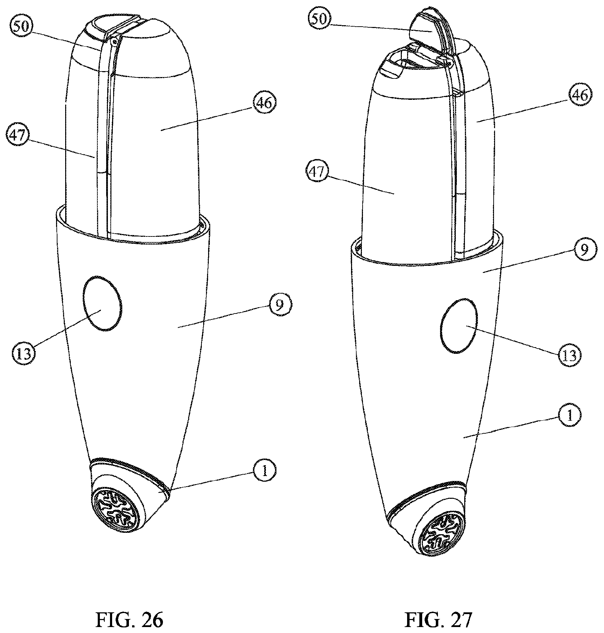

FIG. 2A, FIG. 2B, and FIG. 2C show device 100 with bottom case 52 and transparent case 51 removed, revealing a first container 46 (sometimes referred to herein as a "treatment container") configured to hold a fluid for treating the user's skin ("treatment fluid") and a second container 47 (sometimes referred to herein as a "waste container") in which spent treatment fluid ("waste fluid") is collected. Treatment container 46, in various embodiments, may be configured to be detached from skin cleansing device 100 so that a user can fill treatment container 46 with treatment fluid. Waste container 47, in various embodiments, may be fixedly attached to skin cleansing device 100 and feature a removably attachable end cap 50 (sometimes referred to herein as "waste water tank cover"). Removing end cap 50 may seal an opening in waste container 47 and thus removing end cap 50 may allow a user to drain waste fluid from waste container 47 through the opening and clean the interior of waste container 47. Referring ahead, FIG. 26 and FIG. 27 illustrate an alternative embodiment of removably attachable end cap 50, wherein cap 50 is hinged (shown closed in FIG. 26 and open in FIG. 27).

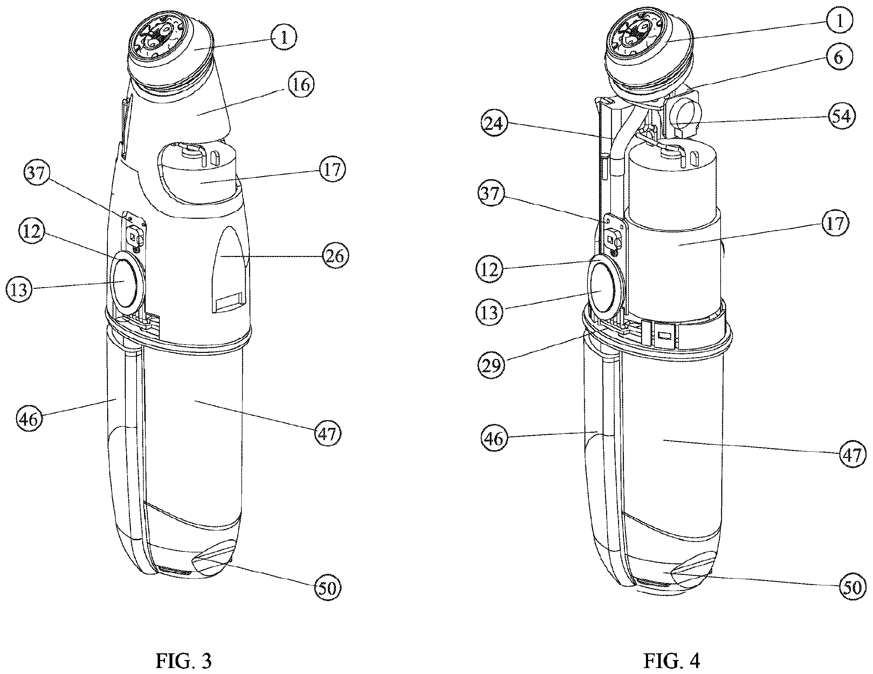

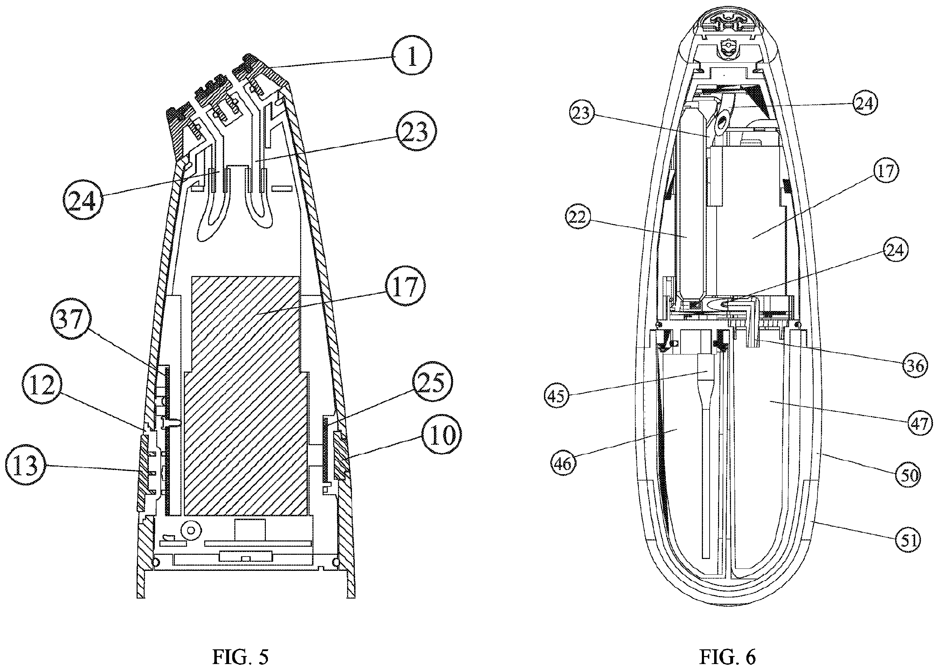

FIG. 3 illustrates handheld skin cleansing device 100 with top case 9 further removed, revealing amongst other miscellaneous components, a pump 17 (sometimes referred to herein as "micro diaphragm vacuum pump"). In operation, pump 17 may be in fluid communication with waste container 47 and configured to form a pressure differential between an interior of waste container 47 and an outer surface of tip 1 so as to draw fluid from treatment container 46, to tip 1, and into waste container 47 via internal conduits, as later described in more detail herein. FIG. 4 illustrates handheld skin cleansing device 100 with pump holders 16 and 26 removed, revealing a first conduit 24 connecting treatment container 46 and tip 1. FIG. 5 is a cutaway view of an upper portion of handheld skin cleansing device 100 comprising tip 1 and pump 17, amongst various other components, and illustrates representative connections of first conduit 24 and a second conduit 23 with tip 1. FIG. 6 is a side view of an interior of handheld skin cleansing device 100 further illustrating such connections. As later described in more detail, a distal end of first conduit 24 may connect with a first opening 1A in tip 1 and thereby serve to deliver treatment fluid to an outer surface 1C of tip 1 from treatment container 46. Likewise, a distal end of second conduit 23 may connect with a second opening 1B in tip 1 and thereby serve to withdraw waste fluid from outer surface 1C of tip 1 and into waste container 47, as later described in more detail. A straw-like conduit 45 (which may, in some cases, be considered an extension of first conduit 24) may extend into an interior of treatment container 46, preferably to a bottom thereof so as to maximize the amount of treatment fluid that can be dispensed from treatment container 46. As shown, transparent case 51 may be positioned such that treatment container 46 and/or waste container 47 are visible to the user when handheld skin cleansing device 100 is fully assembled, thereby allowing a user to easily monitor the levels of fluid in each container 46, 47. In such an embodiment, treatment container 46 and/or waste container 47 may be transparent and, in some embodiments, one or more lights may illuminate containers 46, 47 to enhance visibility and visual effect. An added benefit of such configurations is the ability for the user to immediately see the dirt and other contaminates being removed from his or her skin by the device, providing positive reinforcement. In yet another embodiment, the light(s) may emit ultraviolet light to sterilize the fluid in one or both of the containers, thereby improving the overall hygiene of the device 100.

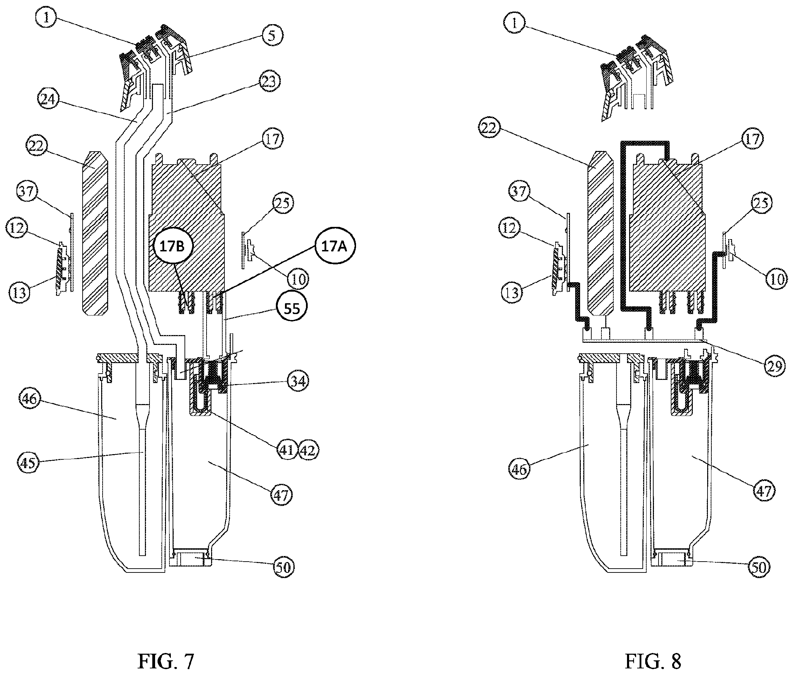

FIG. 7 is a pseudo-schematic view illustrating, amongst other things, fluid connections amongst various components of handheld skin cleansing device 100. In particular, treatment container 46 may be in fluid communication with tip 1 (and in particular, first opening 1A thereof) via first conduit 24 and waste container 47 may be in fluid communication with tip 1 (and in particular, second opening 1B thereof) via second conduit 23. Further, an inflow port 17A of pump 17 may be in fluid communication with waste container 47 via a third conduit 55 and an outflow port 17B of pump 17 may be open to atmosphere. As configured, pump 17 can suck air out of waste container 47 via third conduit 55, disposing of the pumped-out air to atmosphere, and thereby create a pressure differential between the interior of waste container 47 and atmosphere. A similar pressure differential will exist between the interior of waste container 47 and second opening 1B of tip 1, thereby providing a suction force for withdrawing waste fluid from the outer surface of tip 1. As later described in more detail, contacting tip 1 with the user's skin acts as a gate that diverts the circuit to include first conduit 24 and treatment container 46, such that the pressure differential acts to suck treatment fluid from treatment container 46 to first opening 1A of tip 1 for treating the user's skin.

FIG. 8 is a pseudo-schematic view illustrating, amongst other things, electrical connections amongst various components of handheld skin cleansing device 100. In particular, each of PCBA 37 (which controls interface 13), battery 22, pump 17, and PCBA 25 (which controls charging) are in electrical communication with main PCBA 29. As configured, power can be distributed to pump 17 and interface 13 from battery 22 and various features controlled.

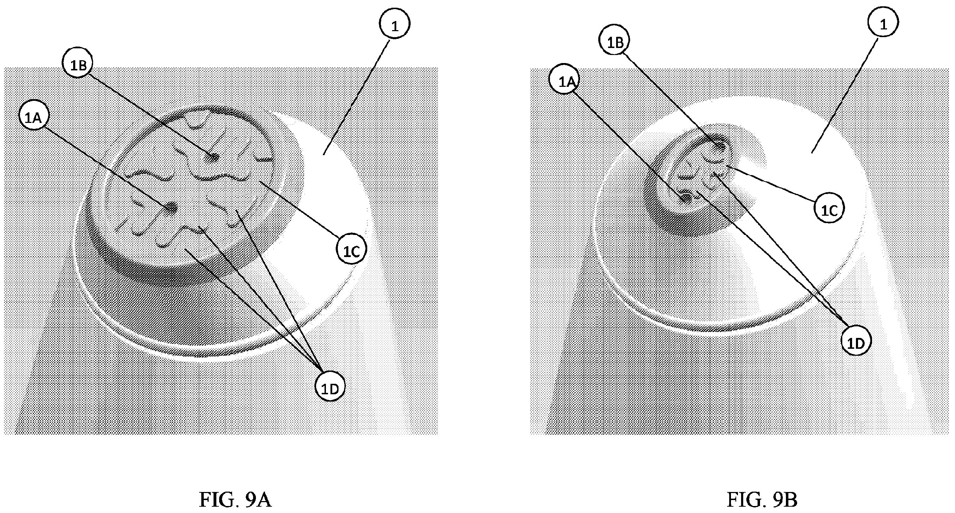

FIG. 9A and FIG. 9B illustrate representative embodiments of tip 1 of handheld skin cleansing device 100. Tip 1, in various embodiments, may comprise an outer surface 1C having a first opening 1A, a second opening 1B, and one or more raised elements 1D extending outwards. First opening 1A may be in fluid communication with first conduit 24 for delivering treatment fluid to outer surface 1C and second opening 1B may be in fluid communication with second conduit 23 for suctioning treatment fluid from outer surface 1C. Raised elements 1D may be configured to create a gap between outer surface 1C and the user's skin for accommodating the treatment fluid, as well as to define pathwaths along outer surface 1C for directing flow of the treatment fluid and generating bubbles, as previously described.

While not shown, in various embodiments, tip 1 and/or the body portion of device 100 may comprise one or more light sources 90 configured to emit light towards the user's skin. In some embodiments, the light source(s) 90 may be on tip 1 while in other additional or alternative embodiments, the light source(s) 90 may be on the main body portion (e.g., top case 9).

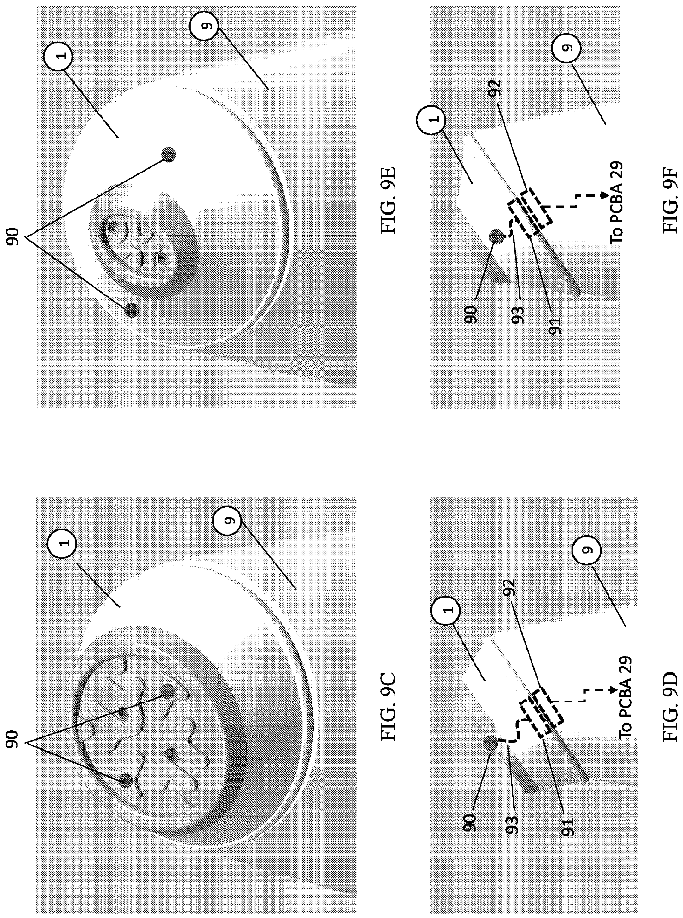

FIG. 9C (iso front view) and FIG. 9D (schematic) illustrate the tip 1 of FIG. 9A with light sources 90 on a front face thereof. FIG. 9E (iso front view) and FIG. 9F (schematic) illustrate the tip 1 of FIG. 9B with light sources 90 on a front face thereof. In those embodiments where light source(s) 90 are on tip 1, the light source(s) may, for example, be (i) positioned on a front face of tip 1 closest to the user's skin in operation (as in FIG. 9A-9F), (ii) embedded within the body of tip 1 (not shown), and/or (iii) positioned on a rear face of tip 1 adjacent the distal end of the main body portion of handheld device 100 (not shown). Regardless of where the light source(s) is located on tip 1, tip 1 may include one or more electrical contacts 91 on the rear surface of tip 1. These electrical contacts 91, in turn, may be positioned to contact complementary electrical contacts 92 on the distal end of the main body portion of handheld device 100 so as to place the tip-located light source(s) 90 into electrical communication with the electronics contained within the main body portion (e.g., PCBA 29) and thereby allow the tip-located light sources 90 to be powered and controlled via the main body portion (e.g., via button 12). In the case of (i) and (ii), tip 1 may further include an electrical conduit(s) 93 (e.g., wire(s)) extending through the body of tip 1 so as to connect light source(s) 90 with the electrical contact 91 on the rear surface of tip 1. In still further embodiments (not shown), tip 1 may include a power source (e.g., battery) to power the light source(s) and a receiver configured to receive functional commands transmitted from electronics in the main body portion of device 100 and thereby provide for controlling the tip-located light source(s) using electronics in the main body portion of device 100.

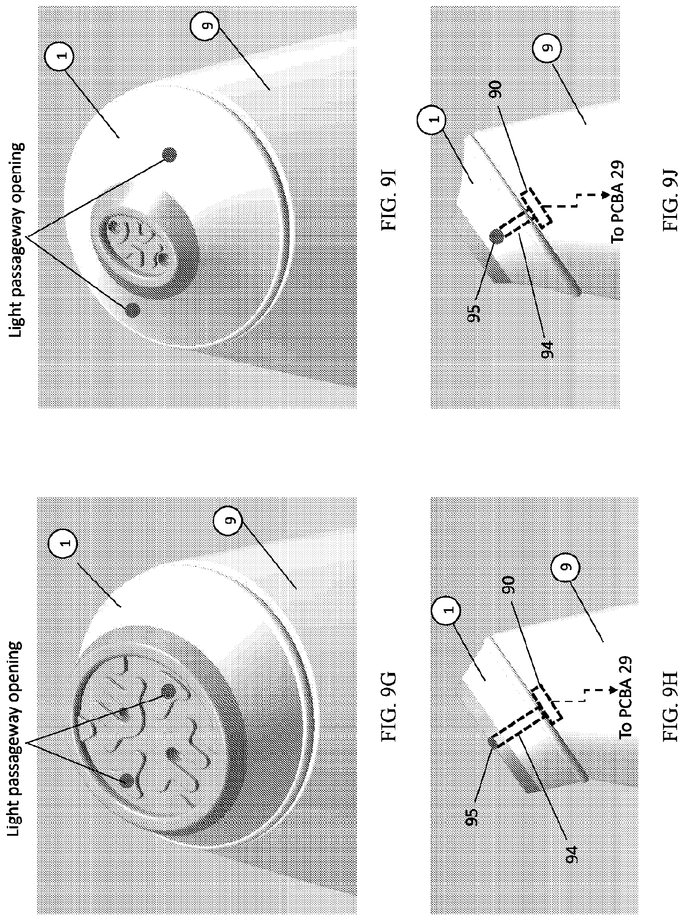

FIG. 9G (iso front view) and FIG. 9H (schematic) illustrate the tip 1 of FIG. 9A with light sources 90 the distal end of the main body portion of device 100 behind tip 1. FIG. 9I (iso front view) and FIG. 9J (schematic) illustrate the tip 1 of FIG. 9B with light sources 90 the distal end of the main body portion of device 100 behind tip 1. In those embodiments where light source(s) 90 are on the main body portion of device 100, light source(s) 90, in various embodiments, may be positioned on a distal end of the main body portion adjacent an underside of tip 1 and configured shine through tip 1 and onto the user's skin in operation. In some embodiments, tip 1 may be made of a material that is sufficiently transparent to allow the light to shine therethrough and onto the skin, such as a clear silicone material. In some other embodiments, tip 1 may comprise one or more optical passageways 94 through tip 1 through which the light can pass. In one such embodiment, the passageway(s) may include a hollow channel(s) aligned with the light source(s) 90 and extending through tip 1 from a rear face to an opening 95 in the front face of tip 1. In another such embodiment, the optical passageway(s) 94 may include a light guide aligned with the light source(s) 90 and extending through tip 1 from a rear face to a front face thereof, such as fiber optics.

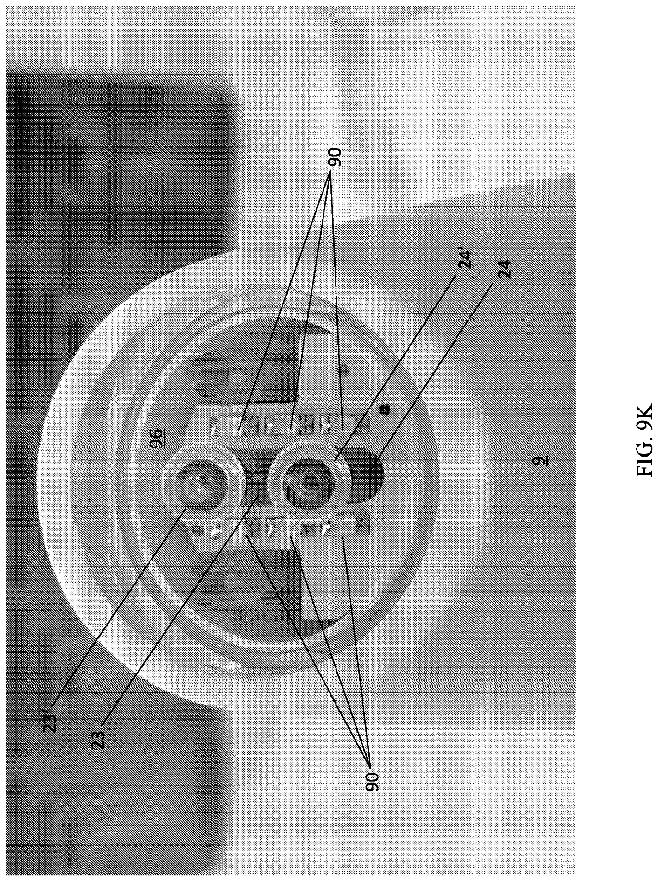

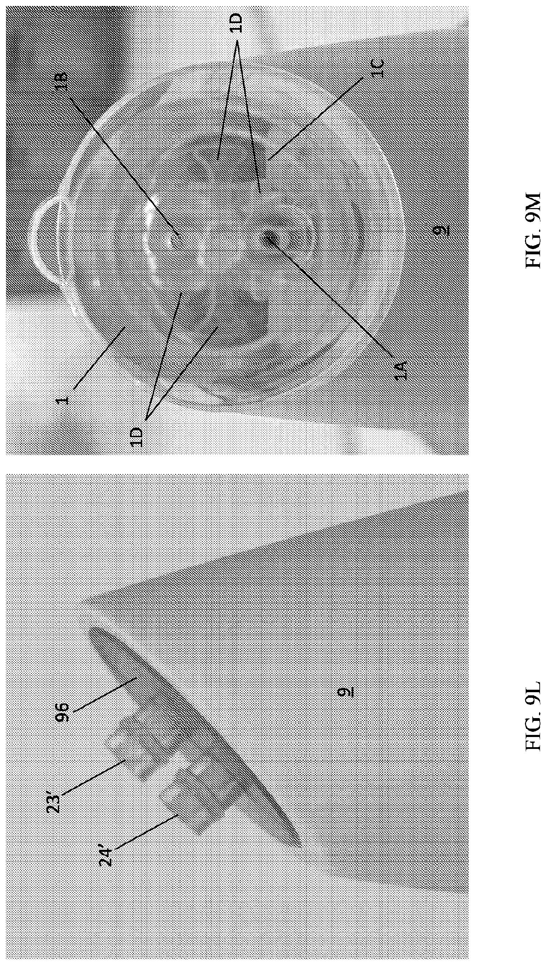

FIG. 9K (front view, tip 1 removed), FIG. 9L (side view, tip 1 removed), and FIG. 9M (front view, transparent tip 1 attached) illustrate a representative configuration in which light source(s) 90 are located at the distal end of the main body portion of device 100 behind tip 1 and where tip 1 is transparent to allow light emitted from the light source(s) 90 to shine through to illuminate the user's skin in operation. Referring first to FIG. 9K, six light sources 90 are located at the distal end of top case 9 proximate where tip 1 attaches. The light sources 90, as well as the hollow interior of top case 9, are covered by a clear plate 96 situated over the large opening at the distal end of top case 9, as shown. As configured, clear plate 96 seals off light sources 90 and the interior of device 100 from light sources 90 from the external environment, thereby protecting each from fluid intrusion, but still allows light emitted from light sources 90 to shine through clear plate 96 towards tip 1 when tip 1 is connected to the distal end of top case 9. As best shown in FIG. 9L and 9M, two conduits 23', 24' extend outwards from clear plate 96 to place conduits 23, 24 into fluid communication with first opening 1B and 1A, respectively, so that treatment fluid can exit and re-enter device 100 through tip 1, respectively, as described herein.

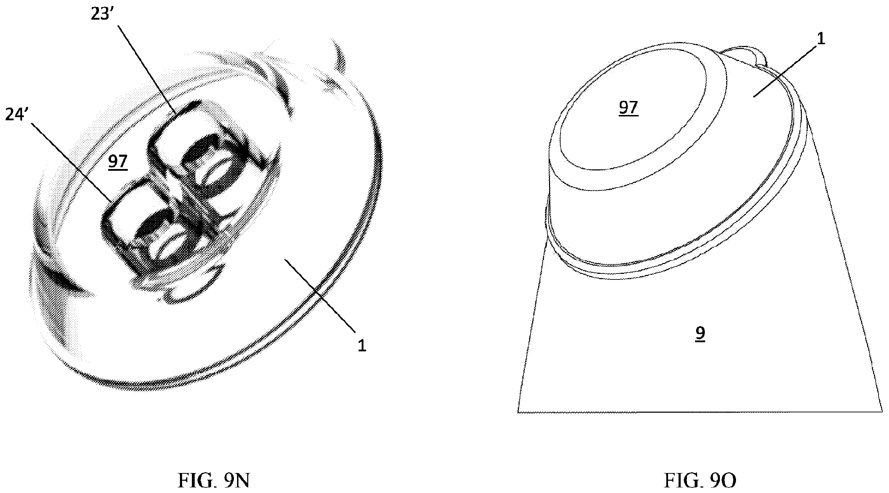

FIG. 9N and FIG. 9O illustrate a representative embodiment of tip 1 configured as a protective cap. Here, tip 1 is not configured as a cleansing tip but rather as a protective cap serving to seal off conduits 23' and 24' when device 100 is not in use. In other words, a user would remove cleansing tip 1 and replace it with protective tip 1 after performing a cleansing treatment. Accordingly, protective tip 1, in various embodiments, may not include openings 1A and 1B but rather just a solid plate 97 so as not to allow fluid to enter device 100 through the distal end of top case 9 or to enter or exit device 100 through conduits 23', 24'. In the embodiment shown, plate 97 is transparent so as to allow light emitted from light sources 90 to shine through plate 97. As configured, a user could use device 100 to perform light-based skin treatments without concurrently performing a cleansing treatment.

Regardless of where the light source(s) are mounted, each may be selectively controlled by the user via interface 13 on the main body portion. In some embodiments, interface 13 may be configured to permit the user to selectively turn on or off one or more of the light sources, whether or not device 100 is in use. For example, a user may be able to turn on one or more of the lights while cleansing the skin with treatment fluid, or turn on one or more of the lights while the cleansing feature is turned off, thereby allowing the user to illuminate the skin for an extended period of time without causing irritation or damage that may otherwise occur were the cleansing feature operating that whole time.

Light source(s), in an embodiment, may emit white light to help the user observe where the tip 1 is on his or her skin in a mirror. Additionally or alternatively, in another embodiment, the light source(s) may be configured to emit light in wavelengths configured to address some skin concerns. For example, red light and blue light in certain wavelengths are known to reduce the appearance of wrinkles, scars, and persistent wounds, and address other skin concerns. The use of such lights in conjunction with fluid cleansing can be particularly effective beyond the sum of the two parts, as opening the pores with fluid cleansing can enhance the effectiveness of the emitted light in addressing certain skin concerns.

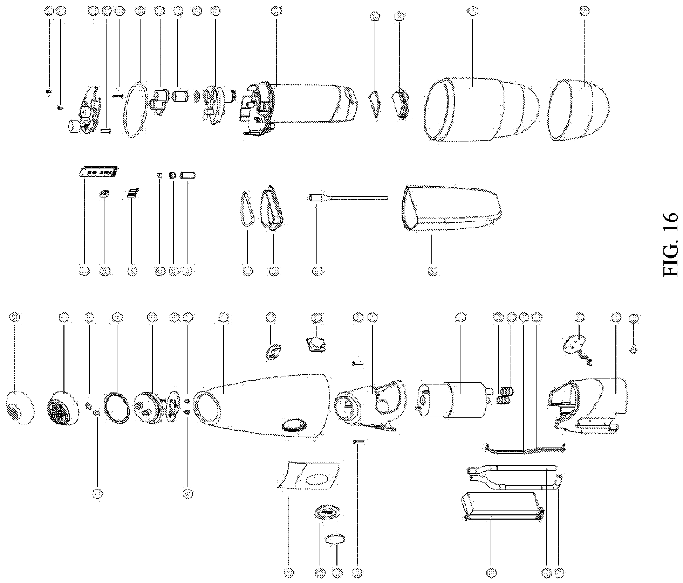

Various other components of handheld skin care device 100 are shown and described in greater detail in the following description and in the exploded view of FIG. 16.

### Operation

In operation, a user may press and hold the "On" button for 2 seconds, the indicator light on the keyboard 37 will light up, and the micro diaphragm vacuum pump 17 will start to operate to form a negative pressure (vacuum). At this time, the suction hole of the micro diaphragm vacuum pump 17 and the waste container 47 will generate a pressure difference. Under this condition, the micro diaphragm vacuum pump 17 suction hole 17A inhales the air from the waste container 47 and discharges by outlet 17B of the micro diaphragm vacuum pump 17.

The micro diaphragm vacuum pump 17 continues to work and the pressure in the waste container 47 decreases slowly. It also forms a pressure difference with the area outside of second opening 1B of tip 1. The air is sucked in from outside to the waste container through the tip 1 and second conduit 23. When tip 1 touches the skin, the device 100 will generate a negative pressure loop. Treatment fluid in treatment container 46 will flow to tip 1 through straw-like conduit 45 and first conduit 24. The suction formed by the negative pressure sucks out impurities and debris from the skin. The treatment fluid flows back and forth in the loop of tip 1, so that the small bubbles carrying the treatment solution can help soften, exfoliate, hydrate, and deliver nutrients to the skin. When the treatment fluid flows to the second opening 1B of tip 1, it is sucked away through second conduit 23 and sucked into the waste container 47. At the same time, it takes away the impurities and debris removed from the skin.

### Pump Protection System 200

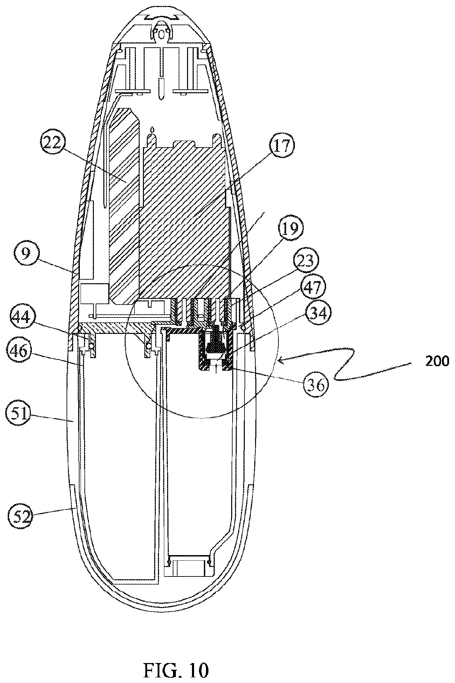

FIG. 10, FIG. 11, and FIG. 12 illustrate a representative embodiment of a pump protection system 200 of the present disclosure. Pump protection system 200, in various embodiments, may be configured to protect pump 17 from ingesting waste fluid from waste container 47 in various embodiments while pump 17 is turned off, as described in more detail herein.

Referring first to FIG. 10, pump protection system 200, in various embodiments, may be situated upstream of pump 17. For example, in the embodiment shown, system 200 is located between waste container 47 and inflow port 17A of pump 17. System 200, in an embodiment, may comprise a check valve 34, which is shown here seated in a portion of waste container middle case 36. Check valve 34, in various embodiments, may comprise a valve housing 34A, a piston 34B, and a spring 34C. Generally speaking, valve housing 34A may be substantially hollow, with a necked opening facing waste container 47. Piston 34B may be situated within valve housing 34A, with its head oriented toward the necked opening of valve housing 34A. Spring 34C may extend along the stem of piston 34B and bias piston 34B towards the necked opening of valve housing 34A.

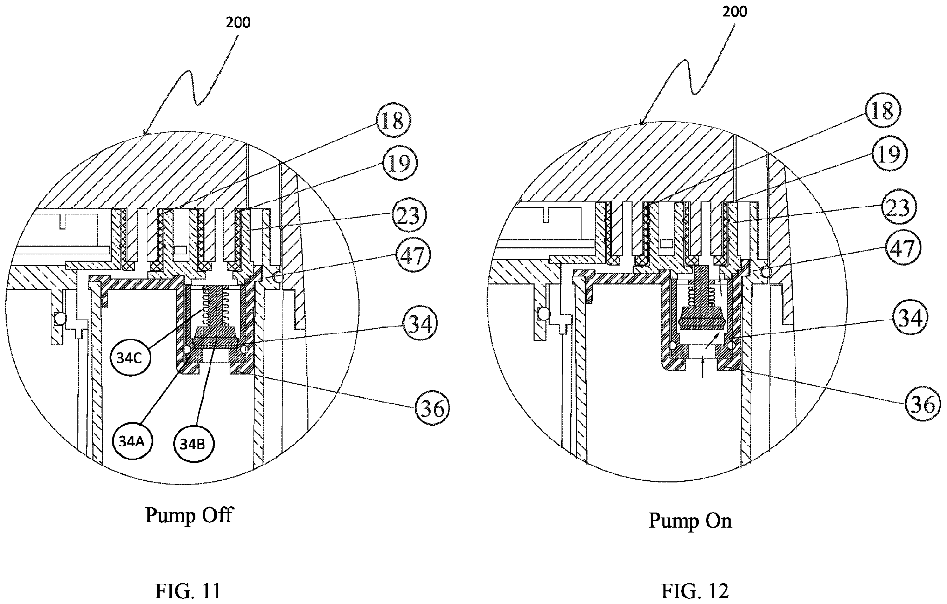

FIG. 11 illustrates a state of pump protection system 200 when pump 17 is turned off. Here, piston 34B is biased against the necked portion of valve housing 34A, sealing off the necked opening and thereby blocking fluid from passing by. FIG. 12 illustrates a state of pump protection system 200 when pump 17 is turned on. Here, suction generated by pump 17 pulls back on piston 34B, causing piston 34B to retract from the necked opening and compressing spring 34C in the process so long as the suction exceeds the spring tension. In this state, pump 17 is in fluid communication with waste container 47, thereby allowing pump 17 to remove air from the interior of waste container 47 and create suction for drawing treatment fluid to tip 1 and associated waste fluid into waste container 47. An inner diameter of the non-necked portion of valve housing 24A may be greater than the outer diameter of the head of piston 24B, such that fluid can pass by piston 24B when piston 24B is retracted from the necked portion. When pump 17 is subsequently turned off, suction ceases and spring 34C pushes the head of piston 34B into the necked portion again, closing check valve 34 to prevent the waste fluid from flowing into pump 17 when device 100 is turned off.

It should be recognized that pump protection 200 is especially useful when coupled included in embodiments where waste container 47 includes a removably attachable end cap 50. In particular, when a user empties waste container 47, he or she may opt to turn device 100 over such that the open end of waste container 47 is pointing up, so as to rinse out the waste container 47 under a faucet. Absent pump protection system 200, faucet water may otherwise flow out of the top of waste container 47, through third conduit 55, and into pump 17 due to gravity. However, since check valve 34 is closed when pump 17 is turned off (which it would be when draining and cleaning waste container 47), check valve 34 prevents water from reaching pump 17.

### Pump Protection System 300

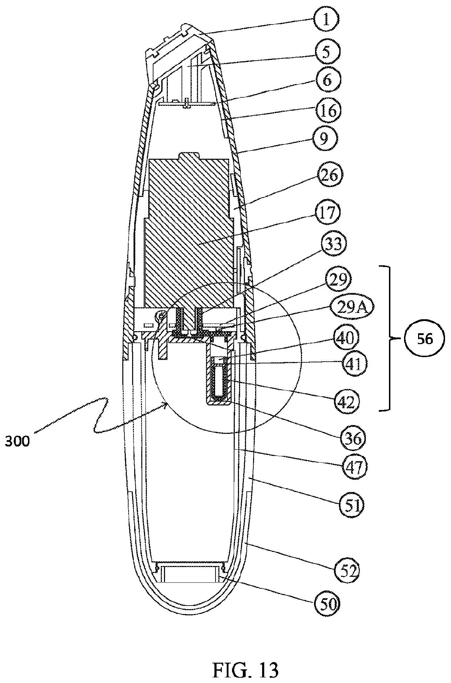

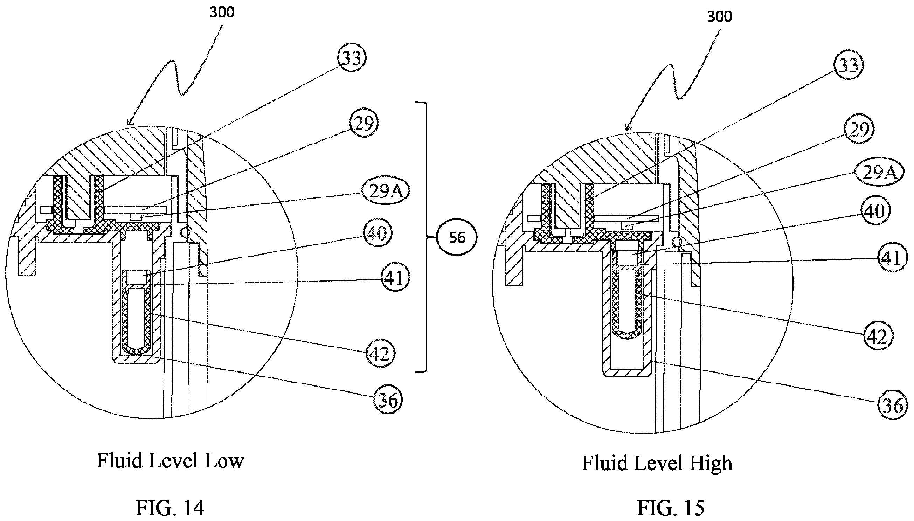

FIG. 13, FIG. 14, and FIG. 15 illustrate a representative embodiment of a pump protection system 300 of the present disclosure. Pump protection system 300, in various embodiments, may be configured to protect pump 17 from ingesting waste fluid from waste container 47 in various embodiments while pump 17 is turned on, such as when waste container 47 is overfilled, as described in more detail herein.

Referring first to FIG. 13, pump protection system 300, in various embodiments, may be situated within or otherwise extend into the top of waste container 47. Here, system 300 is shown as situated within waste container top case 36. System 300, in an embodiment, may comprise a fluid level detection system (FLDS) 56, which is shown here seated in a portion of waste container middle case 36. FLDS 56, in various embodiments, may comprise a float 41/42 having a magnet 40 positioned on top (i.e., on the end of float 41/42 closest to the top of waste container 47), as well as a Hall effect sensor 29A mounted to structure separate from and above float 41/42 and magnet 40. Hall effect sensor 29A may be in electrical communication with main PCBA 29, such that instructions to shut down pump 17 can be transmitted when the Hall effect sensor 29A detects the Hall effect emanating from magnet 40.

Generally speaking, when the waste fluid reaches a certain level in waste container 47, the float 41/42 in the waste container top case 36 will rise slowly. When it reaches the max level (or any other predetermined threshold), magnet 40 on float (41/42) will trigger the Hall effect sensor 29A on the main PCBA 29, and pump 17 will stop working immediately. Device 100 may be configured to automatically sound an alarm until the waste fluid in the waste container 47 is drained. Then, float 41/42 will drop back down towards the bottom end of waste container 47 to a normal position, causing magnet 40 to move away from Hall effect sensor 29A in kind. The micro diaphragm vacuum pump 17 will work again.

Additionally or alternatively, FLDS 56 could be configured to detect when treatment fluid falls below a predetermined level in treatment container 46. In particular, FLDS 56 may turn off the pump 17 upon detecting that fluid in the treatment container 46 reaches a predetermined level corresponding with having dispensed enough treatment fluid for associated waste fluid to reach a predetermined level within the waste container 47. In an embodiment, FLDS 56 could be inverted and situated near the bottom end of treatment container 46 in order to detect low treatment fluid levels. Further, in an embodiment, system 300 may be configured to prevent pump 17 from being turned back on until system 300 detects that waste container 47 has been opened, detached, or otherwise emptied. One of ordinary skill in the art will recognize various ways to detect whether waste container 47 has been opened, detached, or otherwise emptied including, for example, a sensor on end cap 50.

### Pump Protection System 400

While not shown, embodiments of the present disclosure include a pump protection system 400. Similar to pump protection system 300, pump protection system 400 may be configured to protect pump 17 from ingesting waste fluid from waste container 47 in various embodiments while pump 17 is turned on, such as when waste container 47 is overfilled. In particular, system 400 may comprise a moisture sensor upstream of pump 17, such as within third conduit 55. Upon detecting moisture, which would be indicative of waste fluid escaping waste container 47, system 400 may turn off the pump 17 and prevent the pump from being turned back on until detecting that the waste container has been opened, detached, or otherwise emptied.

### Pump Protection System 500

While not shown, embodiments of the present disclosure include a pump protection system 500 which, in various embodiments, may be configured to protect pump 17 from ingesting waste fluid from waste container 47 in various embodiments while pump 17 is turned on and the device 100 is tilted beyond a threshold angle. In particular, an anti-tilt alarm sensor may be added to the main PCBA 29 to remind the user to operate device 100 at a suitable angle. When the anti-tilt alarm sensor senses that the device 100 has tilted beyond a specified angle, pump 17 will stop operation. The device 100 may automatically sound an alarm as well. While the device 100 returns to the proper angle, pump 17 will start operating again.

### Pump Protection System 600

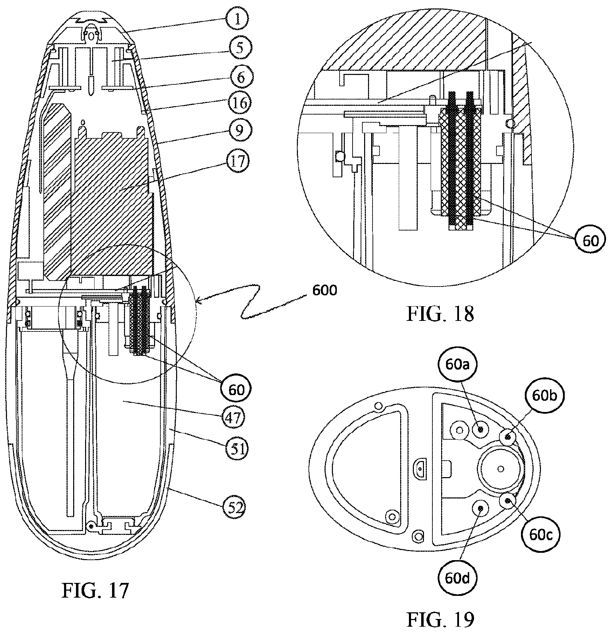

Skipping ahead, FIG. 17, FIG. 18, FIG. 19, FIG. 20A, FIG. 20B, and FIG. 21, illustrate a representative embodiment of a pump protection system 600 of the present disclosure. Similar to pump protection system 300, pump protection system 600, in various embodiments, may be configured to protect pump 17 from ingesting waste fluid from waste container 47 in various embodiments while pump 17 is turned on, such as when waste container 47 is overfilled, as described in more detail herein.

Referring first to FIG. 17 and FIG. 18, pump protection system 600, in various embodiments, may be situated within or otherwise extend into the top of waste container 47. Here, system 600 is shown as situated within waste container top case 36. System 600, in an embodiment, may comprise a plurality of conductive pins 60 in electrical communication with main PCBA 29, such that instructions to shut down pump 17 can be transmitted when waste fluid contacts two or more of pins 60.

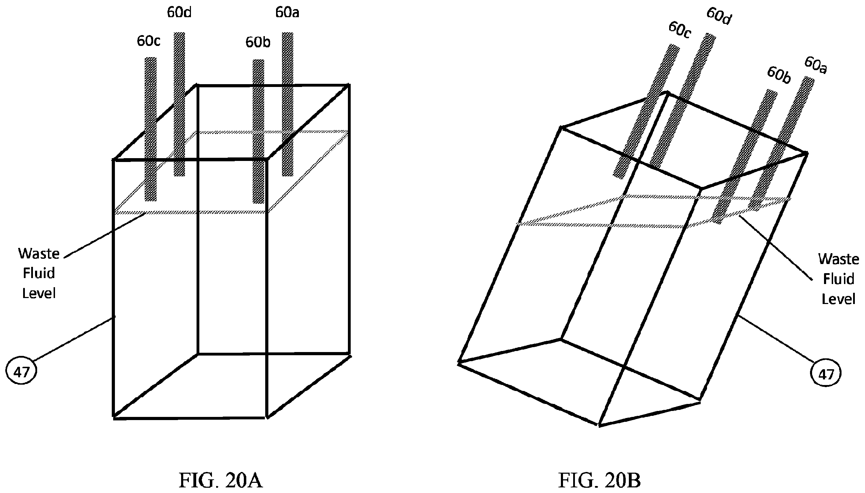

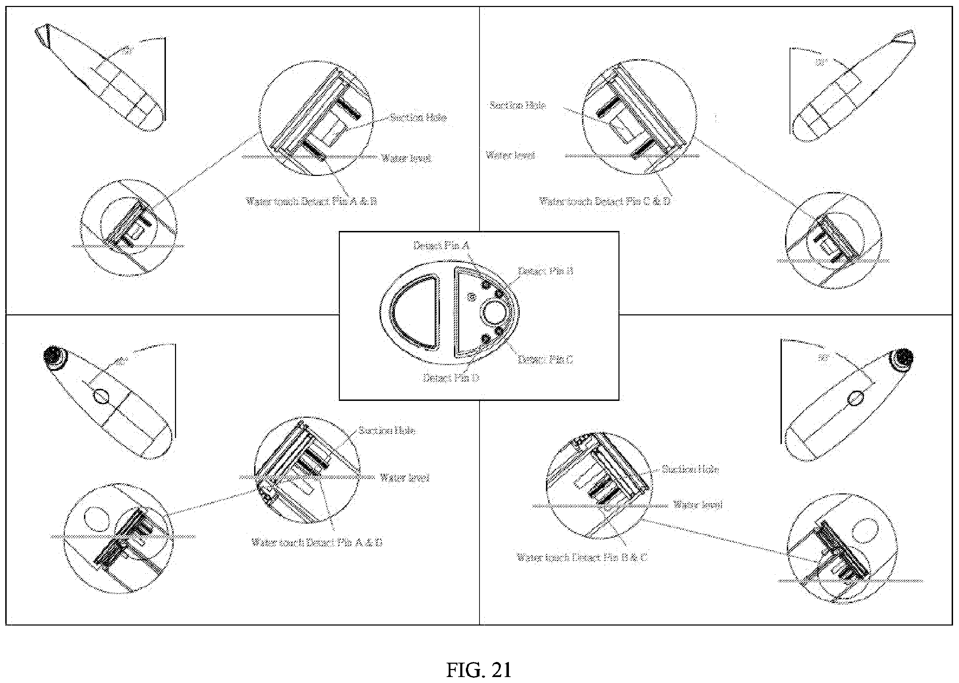

Pins 60, in various embodiments, may be provided in any number and arrangement suitable to detect when the waste fluid reaches a predetermined level regardless of the orientation of device 100 during use. Referring to FIG. 19, system 600 is shown with four pins 60 (i.e., pins 60a, 60b, 60c, and 60d) arranged about an outer perimeter of the interior of waste container 47. With reference to FIG. 20A, FIG. 20B, and FIG. 21, as configured, at least two pins 60 will contact the waste fluid at a predetermined level (i.e., at a level comfortably below where it could be sucked into pump 17) within waste container 47 regardless of whether device 100 is upright or tilted in any particular direction. FIG. 20A shows how, when device 100 is upright, the top of the waste fluid is level within waste container 47 and thereby would contact all four pins 60a, 60b, 60c, 60d upon reaching a height within waste container 47 corresponding with that of the pins 60. FIG. 20B shows how, when device 100 is tilted towards pins 60a, 60b, the top of the waste fluid is sloped within waste container 47 with its highest point in the direction of tilt and, as such, the waste fluid will contact pins 60a, 60b first, and at a height corresponding to the same height within waste container 47 at which the waste fluid contacts all four pins 60 in FIG. 20A when device 100 is upright. While not shown, if device 100 were tilted in the direction of pins 60a, 60d, the waste fluid would first contact pins 60a, 60d at the same height within waste container 47 as in FIG. 20A and FIG. 20B, and likewise if device 100 were tilted in the direction of pins 60c, 60d (with first fluid contact occurring with pins 60c, 60d) or in the direction of pins 60b, 60c (with first fluid contact occurring with pins 60b, 60c).

Upon the waste fluid contacting at least two pins 60, pump 17 will stop working immediately. Device 100 may be configured to automatically sound an alarm until the waste fluid in the waste container 47 is drained, at which point the waste fluid would no longer contact any of pins 60 so long as device 100 is not tilted at an extreme angle (note: device 100 may be shut off anyways in such cases if equipped with the tilt sensor alarm described elsewhere herein). The micro diaphragm vacuum pump 17 will work again.

Additionally or alternatively, system 600 could additionally or alternatively be installed in treatment container 46 and thereby be configured to detect when treatment fluid falls below a predetermined level in treatment container 46. In such a case, pins 60 may extend further down within treatment container 46 to a designated height therewithin corresponding with a low fluid level. When enough fluid has been dispensed, at least some of the pins 60 (the number of which depending on the angle of the device) may no longer contact the fluid, which can be detected and used as an indicator that fluid in the treatment container 46 has reached a predetermined level corresponding with having dispensed enough treatment fluid for associated waste fluid to reach a predetermined level within the waste container 47. System 600 may turn off the pump 17 upon detecting this. In an embodiment, system 600 could be inverted and situated near the bottom end of treatment container 46 in order to detect low treatment fluid levels. Further, in an embodiment, system 600 may be configured to prevent pump 17 from being turned back on until system 600 detects that waste container 47 has been opened, detached, or otherwise emptied. One of ordinary skill in the art will recognize various ways to detect whether waste container 47 has been opened, detached, or otherwise emptied including, for example, a sensor on end cap 50.

It should be recognized that various embodiments of handheld skin cleansing device 100 may comprise any one or combination of pump protection systems 200, 300, 400, 500, and 600, as appropriate.

### Components List

FIG. 16 is an exploded view of the representative skin cleansing device 100 previously described. Table 1 below includes a listing of each component.

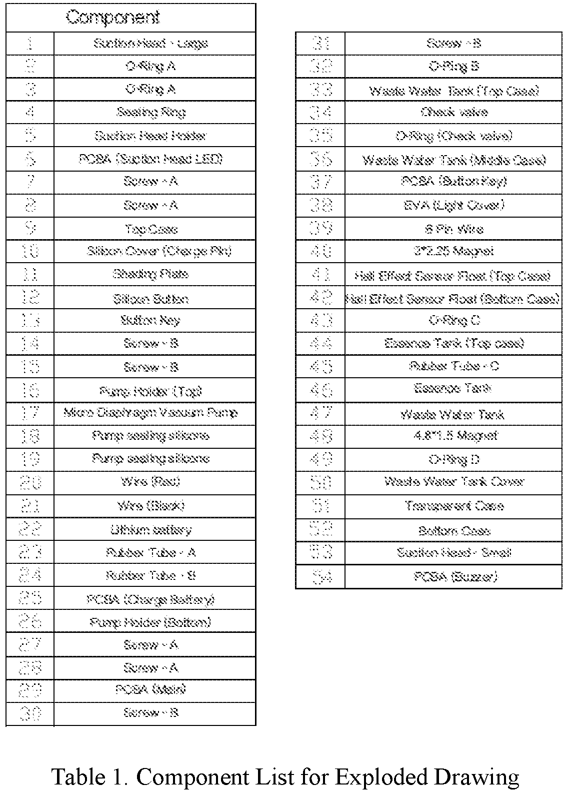

### Head Leakage Valve 70

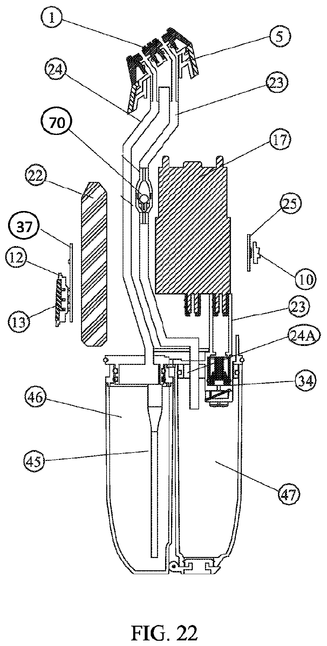

FIG. 22 is a schematic view of device 100 illustrating a head leakage valve 70 positioned within conduit 23 and configured to prevent or mitigate leakage of fluid out of tip 1 when device 100 is inverted for cleaning. For context, when pump 17 is shut off, a pressure differential may remain between the interior of waste container 47 and tip 1, causing fluid that was travelling through conduit 23 at the time pump 17 was shut off to become trapped in conduit 23. This is similar to how one can cover the top of a drinking straw with his or her finger as the straw rests in a glass of water and trap water inside the straw. If the user wishes to empty the waste container thereafter, he or she may open cap 50 from waste container 47. Doing so may equalize the pressure differential and release the forces that had trapped fluid in conduit 23, much like uncovering the end of the straw releases the water trapped in the straw in the drinking water example above. If the user is holding device 100 upside down when he or she opens cap 50, as may be the case since inverting device 100 may make it easier to access the cap 50, then the released fluid may drain out of conduit 23 through tip 1 due to gravity. If a user is not careful to hold device 100 over a sink, the fluid draining out through tip 1 could cause a mess and therefore create an unfavorable user experience. Accordingly, in various embodiments, a conduit 23 may be equipped with a valve 70 configured to obstruct fluid trapped in conduit 23 from leaking out of tip 1 when cap 50 is opened.

Head leakage valve 70, in various embodiments, may be a one-way valve configured to allow fluid to pass therethrough in a direction towards waste container 47, but not allow fluid to pass therethrough in a direction towards tip 1. In various embodiments, locating head leakage valve 70 closer to tip 1 than to waste container 47 can minimize the amount of fluid present within conduit 23 above between head leakage valve 70 and tip 1 at the time pump 17 is shut off and thereby minimize the amount of fluid which may subsequently leak out of tip 1 thereafter. One having ordinary skill in the art will recognize valves suitable to provide the functionality described herein.

### Treatment Fluid Trap 80

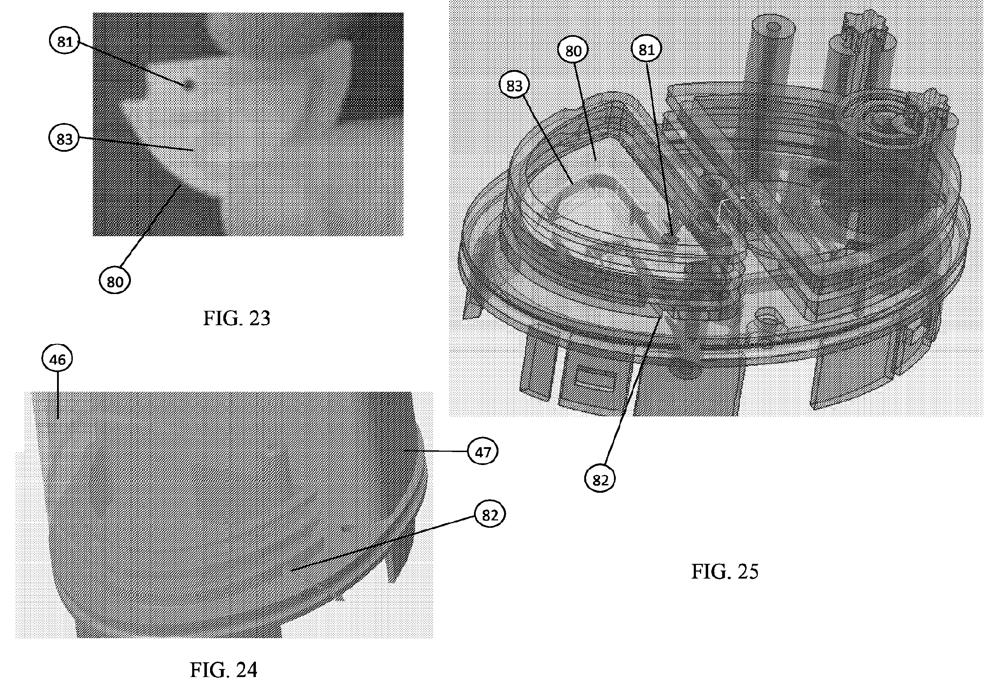

FIG. 23, FIG. 24, and FIG. 25 illustrate a trap 80 configured to prevent or mitigate leakage of treatment fluid out of the top of treatment container 46 when treatment container 46 is being connected to device 100. For context, when treatment container 46 is connected to device 100 (e.g., by inserting the top portion of treatment container 46 into top case 44 for a friction fit or other suitable coupling), a seal may be created early-on during insertion that does not permit air present between the treatment fluid and the top of treatment container 46 to escape. As the top of treatment container 46 is further inserted, the trapped air increases in pressure and in turn pushes downward on the treatment fluid in treatment container 46. The pressure on the treatment fluid may force the treatment fluid up into straw-like conduit 45 where it could, in turn, leak out of tip 1. In an embodiment, a small hole in fluid communication with the interior and exterior of treatment container 46 could be included to relieve this pressure and obviate the leakage issue. However, such a hole may also allow treatment fluid to leak out of treatment container 46 when device 100 is shaken or turned upside down, resulting in leaked fluid collecting inside bottom case 52 of device 100, which may be undesirable from a user experience standpoint. Trap 80, in various embodiments, may be configured to provide pressure relief functionality during connection of treatment container 46 with device 100, while also preventing or mitigating the leakage of treatment fluid out of treatment container 46 when device is shaken or turned upside down.

Trap 80, in various embodiments, may be configured for placement in device 100 where it will place an interior of treatment container 46 in fluid communication with any area outside of the sealed volume created within treatment container 46 upon insertion. As shown in FIG. 25, in an embodiment, such placement may be in top case 46 where the top of treatment container 46 connects to device 100. Trap 80, in various embodiments, may include a body defining a tortuous conduit 83 between a first opening 81 and a second opening 82. In the embodiment shown here, trap 80 is a planar silicone part having a tortuous, hollow channel 83 extending therewithin in a largely planar direction between a first opening 81 through an upper surface of the part and a second opening through a side surface of the part. When assembled, first opening 81 may be positioned so as to be in fluid communication with an interior of treatment container 46 and second opening 82 may be positioned so as to open into the area outside of the sealed volume within treatment container upon insertion - here, into an area within bottom case 52.

By placing an interior of treatment container 46 in fluid communication with an external volume, air within treatment container 46 that may be pressurized during connection of treatment container 46 to device 100 can escape through tortuous conduit 83 as shown in FIG. 25 and thereby not force treatment fluid into straw-like conduit 45. While trap 80 may allow treatment fluid to drain into tortuous conduit 83 through first opening 81, the tortuous pathway defined by the shape of tortuous conduit 83 may serve to become "trapped" within tortuous conduit 83 and thereby not escape into bottom case 52 through second opening 82. As used herein, the term "tortuous" means a pathway defining at least one bend or turn such that the pathway would have to be tilted in various directions in a predetermined sequence in order for gravity to pull fluid therethrough and out of second opening 82. One having ordinary skill in the art will recognize various tortuous pathways suitable to provide the functionality described herein.

Although the present disclosure and its advantages have been described in detail, it should be understood that various changes, substitutions and alterations can be made herein without departing from the scope of the disclosure as defined by the appended claims.

## Claims

1. A handheld skin cleansing device (100) comprising:
a first conduit (24) providing fluid communication between a treatment container (46) and a first opening in a tip (1) of the device:
a second conduit (23) providing fluid communication between a second opening in the tip (1) and a waste container (47) of the device, the waste container (47) being fixedly attached to a handheld body of the device and having an opening sealed by removable cap (50);
a pump (17) housed within the handheld body and in fluid communication with the waste container (47), the pump (17) configured to form a pressure differential between the waste container (47) and the second opening in the tip (1) so as to direct fluid from the treatment container (46), to the tip (1), and into the waste container (47) via the first and second conduits (24, 23) when the tip (1) is applied to skin of a user; and
a valve (34) configured to open to permit fluid communication between the waste container (47) and the pump (17) in response to suction forces generated by operation of the pump (17), and to close to obstruct fluid communication between the waste container (47) and the pump (17) in the absence of the suction forces when the pump (17) is not being operated.

2. The handheld skin cleansing device (100) of claim 1, wherein the valve (34) comprises:
a piston (34B) configured to move between a first position and a second position and wherein, in the first position, the piston (34B) does not obstruct fluid communication between the waste container (47) and the pump (17) and wherein, in the second position, the piston (34B) obstructs fluid communication between the waste container (47) and the pump (17); and
a biasing member (34C) coupled to the piston (34B), the biasing member (34C) being configured to permit the piston (34B) to move to the first position in response to the suction forces generated by operation of the pump (17), and to bias the piston (34B) into the second position in the absence of the suction forces when the pump (17) is not being operated.

3. The handheld skin cleansing device (100) of claim 1,
wherein the removable cap (50) is configured to be opened to allow the waste fluid to be drained from the waste container (47) through the opening and to introduce a cleaning fluid into the waste container (47), and
wherein the valve (34) closes in the absence of the suction forces when the pump (17) is not being operated, thereby allowing the device to be inverted to facilitate the introduction of the cleaning fluid into the waste container (47) through the opening without the cleaning fluid draining into the pump (17).

4. The handheld skin cleansing device (100) of claim 1, further comprising a valve (34) situated within the second conduit (23), the valve (34) configured to obstruct the flow of fluid therethrough in a direction towards the tip (1) when a pressure differential across the second conduit (23) is released.

5. The handheld skin cleansing device (100) of claim 1, further comprising a pump protection system (200, 300, 400, 500, 600) configured to turn the pump off upon detecting any one or more of the following: (i) that fluid in the waste container (47) reaches a predetermined level, (ii) that fluid in the treatment container (46) reaches a predetermined level corresponding with having dispensed enough fluid for the fluid to reach a predetermined level within the waste container (47), and (iii) that there is moisture upstream of the pump (17).

6. The handheld skin cleansing device of claim 5, wherein the pump protection system (300) comprises a hall effect float sensor (29A).

7. The handheld skin cleansing device (100) of claim 5, wherein the pump protection system (600) comprises a sensor having three or more conductive pins (60) extending an equal distance into the waste container (47) and arranged about a perimeter of an interior of the waste container (47), the pin (60) being configured to detect when waste fluid contacts at least two of the pins (60) simultaneously.

8. The handheld skin cleansing device (100) of claim 1, further comprising a pump protection system (500) configured to detect that an angle of the device has exceeded a predetermined threshold and to turn off the pump (17) in response.

9. The handheld skin cleansing device (100) of claim 8, wherein the pump protection system (500) comprises a tilt angle sensor.

10. The handheld skin cleansing device (100) of claim 1, further comprising a trap (80) having a tortuous conduit (83) extending between a first opening (81) and a second opening (82), the first opening (81) being in fluid communication with an interior of the treatment container (46) and the second opening (82) being in fluid communication with an area outside of the treatment container (46).

## Patentansprüche

1. Tragbare Hautreinigungsvorrichtung (100), Folgendes umfassend:
eine erste Leitung (24), die eine Fluidkommunikation zwischen einem Behandlungsbehälter (46) und einer ersten Öffnung in einer Spitze (1) der Vorrichtung bereitstellt;
eine zweite Leitung (23), die eine Fluidkommunikation zwischen einer zweiten Öffnung in der Spitze (1) und einem Abfallbehälter (47) der Vorrichtung bereitstellt, wobei der Abfallbehälter (47) fest an einem tragbaren Körper der Vorrichtung befestigt ist und eine durch die abnehmbare Kappe (50) verschlossene Öffnung aufweist;
eine Pumpe (17), die innerhalb des tragbaren Körpers untergebracht ist und in Fluidkommunikation mit dem Abfallbehälter (47) steht, wobei die Pumpe (17) konfiguriert ist, eine Druckdifferenz zwischen dem Abfallbehälter (47) und der zweiten Öffnung in der Spitze (1) zu bilden, um über die erste und die zweite Leitung (24, 23) Fluid aus dem Behandlungsbehälter (46) zu der Spitze (1) und in den Abfallbehälter (47) zu leiten, wenn die Spitze (1) auf die Haut eines Benutzers aufgebracht wird; und
ein Ventil (34), das konfiguriert ist, sich zu öffnen, um eine Fluidkommunikation zwischen dem Abfallbehälter (47) und der Pumpe (17) als Reaktion auf Saugkräfte zu ermöglichen, die durch den Betrieb der Pumpe (17) erzeugt werden, und sich zu schließen, um die Fluidkommunikation zwischen dem Abfallbehälter (47) und der Pumpe (17) bei Abwesenheit der Saugkräfte zu versperren, wenn die Pumpe (17) nicht betrieben wird.

2. Tragbare Hautreinigungsvorrichtung (100) nach Anspruch 1, wobei das Ventil (34) Folgendes umfasst:
einen Kolben (34B), der konfiguriert ist, sich zwischen einer ersten Position und einer zweiten Position zu bewegen und wobei in der ersten Position der Kolben (34B) die Fluidkommunikation zwischen dem Abfallbehälter (47) und der Pumpe (17) nicht versperrt und wobei in der zweiten Position der Kolben (34B) die Fluidkommunikation zwischen dem Abfallbehälter (47) und der Pumpe (17) versperrt; und
ein Vorspannelement (34C), das mit dem Kolben (34B) gekoppelt ist, wobei das Vorspannelement (34C) konfiguriert ist, dem Kolben (34B) zu ermöglichen, sich als Reaktion auf die durch den Betrieb der Pumpe (17) erzeugten Saugkräfte in die erste Position zu bewegen, und den Kolben (34B) bei Abwesenheit der Saugkräfte in die zweite Position vorzuspannen, wenn die Pumpe (17) nicht betrieben wird.

3. Tragbare Hautreinigungsvorrichtung (100) nach Anspruch 1, wobei die abnehmbare Kappe (50) konfiguriert ist, geöffnet zu werden, um das Abfallfluid durch die Öffnung aus dem Abfallbehälter (47) abzulassen und ein Reinigungsfluid in den Abfallbehälter (47) einzubringen, und
wobei sich das Ventil (34) bei Abwesenheit der Saugkräfte schließt, wenn die Pumpe (17) nicht betrieben wird, wodurch die Vorrichtung umgedreht werden kann, um das Einbringen des Reinigungsfluids in den Abfallbehälter (47) durch die Öffnung zu erleichtern, ohne dass das Reinigungsfluid in die Pumpe (17) abgelassen wird.

4. Tragbare Hautreinigungsvorrichtung (100) nach Anspruch 1, ferner ein Ventil (34) umfassend, das sich innerhalb der zweiten Leitung (23) befindet, wobei das Ventil (34) konfiguriert ist, den Fluss von Fluid durch diese in Richtung der Spitze (1) zu versperren, wenn eine Druckdifferenz über die zweite Leitung (23) abgebaut wird.

5. Tragbare Hautreinigungsvorrichtung (100) nach Anspruch 1, ferner ein Pumpenschutzsystem (200, 300, 400, 500, 600) umfassend, das konfiguriert ist, die Pumpe abzuschalten, wenn eines oder mehrere von Folgendem erkannt werden: (i) dass das Fluid in dem Abfallbehälter (47) einen vordefinierten Pegel erreicht, (ii) dass das Fluid in dem Behandlungsbehälter (46) einen vordefinierten Pegel erreicht, was dem entspricht, dass genügend Fluid abgegeben wurde, damit das Fluid einen vordefinierten Pegel innerhalb des Abfallbehälters (47) erreicht, und (iii) dass Feuchtigkeit stromaufwärts der Pumpe (17) vorhanden ist.

6. Tragbare Hautreinigungsvorrichtung nach Anspruch 5, wobei das Pumpenschutzsystem (300) einen Hall-Effekt-Schwimmersensor (29A) umfasst.

7. Tragbare Hautreinigungsvorrichtung (100) nach Anspruch 5, wobei das Pumpenschutzsystem (600) einen Sensor umfasst, der drei oder mehr leitfähige Stifte (60) aufweist, die sich um denselben Abstand in den Abfallbehälter (47) erstrecken und um den Umfang des Inneren des Abfallbehälters (47) angeordnet sind, wobei der Stift (60) konfiguriert ist zu erkennen, wenn Abfallfluid gleichzeitig mindestens zwei der Stifte (60) berührt.

8. Tragbare Hautreinigungsvorrichtung (100) nach Anspruch 1, ferner ein Pumpenschutzsystem (500) umfassend, das konfiguriert ist zu erkennen, dass ein Winkel der Vorrichtung einen vorbestimmten Schwellenwert überschritten hat, und als Reaktion darauf die Pumpe (17) abzuschalten.

9. Tragbare Hautreinigungsvorrichtung (100) nach Anspruch 8, wobei das Pumpenschutzsystem (500) einen Neigungswinkelsensor umfasst.

10. Tragbare Hautreinigungsvorrichtung (100) nach Anspruch 1, ferner einen Rückhalter (80) umfassend, der eine gewundene Leitung (83) aufweist, die sich zwischen einer ersten Öffnung (81) und einer zweiten Öffnung (82) erstreckt, wobei die erste Öffnung (81) in Fluidkommunikation mit dem Inneren des Behandlungsbehälters (46) steht und die zweite Öffnung (82) in Fluidkommunikation mit dem Bereich außerhalb des Behandlungsbehälters (46) steht.

## Revendications

1. Dispositif de nettoyage de la peau portatif (100) comprenant :
un premier conduit (24) assurant une communication fluidique entre un récipient de traitement (46) et une première ouverture dans un embout (1) du dispositif ;
un second conduit (23) assurant une communication fluidique entre une seconde ouverture dans l'embout (1) et un récipient de récupération (47) du dispositif, le récipient de récupération (47) étant fixé à demeure à un corps portatif du dispositif et présentant une ouverture fermée hermétiquement par un capuchon amovible (50) ;
une pompe (17) logée à l'intérieur du corps portatif et en communication fluidique avec le récipient de récupération (47), la pompe (17) étant configurée pour former un différentiel de pression entre le récipient de récupération (47) et la seconde ouverture dans l'embout (1) de manière à diriger du fluide depuis le récipient de traitement (46), vers l'embout (1), et dans le récipient de récupération (47) par l'intermédiaire des premier et second conduits (24, 23) lorsque l'embout (1) est appliqué sur la peau d'un utilisateur ; et
une vanne (34) configurée pour s'ouvrir afin de permettre une communication fluidique entre le récipient de récupération (47) et la pompe (17) en réponse à des forces d'aspiration générées par le fonctionnement de la pompe (17), et pour se fermer afin d'obstruer la communication fluidique entre le récipient de récupération (47) et la pompe (17) en l'absence des forces d'aspiration lorsque la pompe (17) ne fonctionne pas.

2. Dispositif de nettoyage de la peau portatif (100) selon la revendication 1, dans lequel la vanne (34) comprend :
un piston (34B) configuré pour se déplacer entre une première position et une seconde position et dans lequel, dans la première position, le piston (34B) n'obstrue pas la communication fluidique entre le récipient de récupération (47) et la pompe (17) et dans lequel, dans la seconde position, le piston (34B) obstrue la communication fluidique entre le récipient de récupération (47) et la pompe (17) ; et
un élément de rappel (34C) couplé au piston (34B), l'élément de rappel (34C) étant configuré pour permettre au piston (34B) de se déplacer vers la première position en réponse aux forces d'aspiration générées par le fonctionnement de la pompe (17), et pour rappeler le piston (34B) dans la seconde position en l'absence des forces d'aspiration lorsque la pompe (17) ne fonctionne pas.

3. Dispositif de nettoyage de la peau portatif (100) selon la revendication 1, dans lequel le capuchon amovible (50) est configuré pour être ouvert pour permettre l'évacuation du fluide résiduaire hors du récipient de récupération (47) à travers l'ouverture et pour introduire un fluide de nettoyage dans le récipient de récupération (47), et
dans lequel la vanne (34) se ferme en l'absence des forces d'aspiration lorsque la pompe (17) ne fonctionne pas, permettant ainsi une inversion du dispositif pour faciliter l'introduction du fluide de nettoyage dans le récipient de récupération (47) à travers l'ouverture sans écoulement du fluide de nettoyage dans la pompe (17).

4. Dispositif de nettoyage de la peau portatif (100) selon la revendication 1, comprenant en outre une vanne (34) située à l'intérieur du second conduit (23), la vanne (34) étant configurée pour obstruer l'écoulement de fluide à travers celle-ci dans une direction allant vers l'embout (1) lorsqu'un différentiel de pression à travers le second conduit (23) est relâché.

5. Dispositif de nettoyage de la peau portatif (100) selon la revendication 1, comprenant en outre un système de protection de pompe (200, 300, 400, 500, 600) configuré pour arrêter la pompe lors de la détection de l'un quelconque ou de plusieurs des éléments suivants : (i) l'atteinte d'un niveau prédéterminé par le fluide dans le récipient de récupération (47), (ii) l'atteinte d'un niveau prédéterminé par le fluide dans le récipient de traitement (46) correspondant à la distribution d'une quantité suffisante de fluide pour que le fluide atteigne un niveau prédéterminé à l'intérieur du récipient de récupération (47), et (iii) la présence d'humidité en amont de la pompe (17).

6. Dispositif de nettoyage de la peau portatif selon la revendication 5, dans lequel le système de protection de pompe (300) comprend un capteur à flotteur à effet Hall (29A).

7. Dispositif de nettoyage de la peau portatif (100) selon la revendication 5, dans lequel le système de protection de pompe (600) comprend un capteur présentant trois broches conductrices (60) ou plus s'étendant sur une distance égale dans le récipient de récupération (47) et agencées autour d'un périmètre d'un intérieur du récipient de récupération (47), la broche (60) étant configurée pour détecter le contact simultané du fluide résiduaire avec au moins deux des broches (60).

8. Dispositif de nettoyage de la peau portatif (100) selon la revendication 1, comprenant en outre un système de protection de pompe (500) configuré pour détecter le dépassement d'un seuil prédéterminé par un angle du dispositif et pour arrêter la pompe (17) en réponse.

9. Dispositif de nettoyage de la peau portatif (100) selon la revendication 8, dans lequel le système de protection de pompe (500) comprend un capteur d'angle d'inclinaison.

10. Dispositif de nettoyage de la peau portatif (100) selon la revendication 1, comprenant en outre un piège (80) présentant un conduit sinueux (83) s'étendant entre une première ouverture (81) et une seconde ouverture (82), la première ouverture (81) étant en communication fluidique avec un intérieur du récipient de traitement (46) et la seconde ouverture (82) étant en communication fluidique avec une zone extérieure au récipient de traitement (46).
